# EUROPEAN PATENT APPLICATION

(11) **EP 4 574 966 A1**
(43) Date of publication of application: **25.06.2025**
(21) Application number: 24221186.0
(22) Date of filing: 18.12.2024
(51) Int. Cl.: C12N 5/077, C12N 9/04, C12N 9/12

(54) **ENGINEERED CELL LINES FOR THE PRODUCTION OF CULTIVATED MEAT**

(30) Priority: 18.12.2023 US 202363611589 P
(71) Applicant: Good Meat, Inc., Alameda, CA 94501 (US)
(72) Inventor: SANTO, Vitor Espirito, Alameda, 94501 (US); YANG, Chih Chao, Alameda, 94501 (US); NGUYEN, Chuong, Alameda, 94501 (US)
(74) Representative: EIP

(57) **Abstract**

Cell lines that can be utilized in fed-batch processes are provided. In preferred embodiments, the cell lines have been modified to reduce or eliminate the production of byproduct(s) that adversely impact cell proliferation and/or viability in fed-batch processes. In some embodiments, the cells are modified by inactivating one or more genes that encode for lactate production. Exemplary genes include lactate dehydrogenase A (LDHA) and pyruvate dehydrogenase kinase 1 (PDK1). Also provided are methods of making and using the cells, for example in food products.

## Description

### Priority Claim

The present application claims the benefit of priority from U.S. Provisional Application No. 63/611,589, filed on December 18, 2023, the entirety of which is incorporated herein by reference.

### Field

Cell lines that have been genetically modified to reduce or eliminate the production of by-products that adversely impact cell proliferation and/or viability, methods of making such cell lines, and methods of using such cell lines to produce cultivated meat are described herein.

### Background

Cultured or cultivated meat involves the direct culturing of animal cells to produce food products. Cultivated meat has the potential to address the environmental, ethical, and public-health concerns associated with traditional slaughtered meat.

Cultivated meat is typically manufactured by acquiring and banking stem cells from a desired animal (e.g., chicken, cow, fish, etc.). The cells are grown in bioreactors (known colloquially as cultivators) to achieve high densities and volumes. Similar to what happens inside an animal's body, the cells are fed an oxygen-rich cell culture medium made up of basic nutrients such as amino acids, glucose, vitamins, and inorganic salts, and supplemented with growth factors and other proteins to reach the required densities and volumes.

In some scenarios, the cells used are immature cells. In order to produce the necessary cell types, changes in the medium composition, often in tandem with cues from a scaffolding structure, can trigger the immature cells to differentiate into the skeletal muscle, fat, and connective tissues that make up meat. The cells can then be harvested, prepared, and packaged into final products.

A variety of techniques are known in the art to culture and proliferate cells. "Continuous cell culture" involves the continuous addition of cell culture medium to remove nutrients and metabolic end products. While continuous cell culture allows for the maintenance of a constant phase of cell growth, the requirement of the continuous addition of media can make such processes expensive and labor intensive. This is more acute at the degree of scale-up needed for the commercial production of cultivated meat for food service and retail (e.g., grocery stores, restaurants, etc.) applications.

Alternative methods for culturing cells include batch and fed-batch processes. In a batch process, all nutrients are provided at the beginning of the cultivation; no additional nutrients are added - just control elements such as gases, acids, and bases. It is a closed system; the batch process lasts until the nutrients are consumed.

Batch processes are typically most suitable for rapid experiments such as strain characterization or the optimization of nutrient medium. A disadvantage of this method is that the biomass and product yields are limited. Since the carbon source and/or oxygen transfer are usually the limiting factor(s), the cells are not in the exponential growth phase for long periods time.

One way of keeping nutrients from becoming a limiting factor in cell proliferation is to constantly supply them during proliferation. This is called a fed-batch process, which is a partly open system. The advantage of feeding during cultivation is that it allows one to achieve overall higher product quantities. Under specific growth conditions, the cells typically constantly double and therefore follow an exponential growth curve. The user can either manually set the feed at any time (linear, exponential, pulse-wise), or add nutrients when specific conditions are met, such as when a certain cell concentration is reached or when a nutrient is depleted. While the batch process is classified as a discontinuous process, a fed-batch process is a semi-continuous process.

The fed-batch process offers a wide range of control strategies and is also suitable for highly specialized applications. The fed-batch process is significantly less expensive than the continuous process since it does not require the continuous replacement of cell culture media and the labor time and expense associated with this replacement. However, fed-batch processes may increase the processing time to reach the desired cell densities and lead to inhibition of cell proliferation through the accumulation of by-products that adversely affect cell growth and thus viable cell densities (VCDs).

Therefore, there exists a need for cell lines that can be utilized in economical fed-batch processes to achieve the necessary cell densities/viabilities for the commercial production of cultivated/hybrid meat.

There also exists a need for cell lines that have been modified to reduce or eliminate the production of by-products that adversely impact cell proliferation and/or viability in fed-batch processes, particularly at commercial scale.

### Summary

Thus, it is an object of the invention to provide cell lines that can be utilized in economical fed-batch processes to achieve the necessary cell densities/viabilities for the commercial production of cultivated/hybrid meat. It is also an object of the invention to cell lines that have been modified to reduce or eliminate the production of by-products that adversely impact cell proliferation and/or viability in fed-batch processes, particularly at commercial scale. It is also an object of the invention to provide methods of making and using the cells, for example in food products.

One or more cell lines, which has been modified to affect the production of by-products that adversely affect cell proliferation in the production of cultivated/hybrid meat, and methods of production and use thereof, are described herein.

In some embodiments, the one or more cell lines are mammalian cell lines. In some embodiments, the one or more cell lines are avian cell lines (e.g., chicken, duck, turkey, quail, pheasant, goose, etc.). In some embodiments, the one or more cell lines are bovidae cell lines (e.g., cows, sheep, goats, etc.). In some embodiments, the one or more cell lines are fish cell lines, such as Osteichthyes (bony fish). In some embodiments, the one or more cell lines are derived from other families, classes, or species.

The cell lines described herein can be any type of cell. In some embodiments, the cell type is suitable for use in the production of cultivated meat. In some embodiments, the cells are fibroblasts, muscle cells, myosatellite cells, myoblasts, pre-adipocytes, adipocytes, epithelial cells, or combinations thereof, of animals that are adapted for cultivation in an appropriate growth medium.

In some embodiments, the cell lines are modified or manipulated to inactivate or knock out one or more genes involved in, or responsible for, the production of one or more molecules (e.g., metabolites or metabolic waste product) that adversely affect or impact cell proliferation and/or viability. In some embodiments, the one or more molecules is lactate. In some embodiments, the one or more genes that are inactivated or knocked out are lactate dehydrogenase A (LDHA), pyruvate dehydrogenase kinase 1 (PDK1) and combinations thereof. In some embodiments, the one or more genes that are inactivated or knocked out are LDHA and PDK1. In some embodiments, the resulting lactate concentration in the media after genetic modification is reduced by at least 50%. In some embodiments, the lactate concentration is reduced by at least 25%, 30%, 35%, 40%, 45%, 50%, 55%, 60%, 65%, 70%, 75%, 80%, 85%, 90%, or 95% compared to the unmodified cell lines. The reduction can be, for example, compared to an equivalent number of cells without the inactivation or knockout of the target gene(s) (e.g., LDHA, PDK1, or combination thereof), also referred to herein as unmodified cells, cultured under the same conditions.

In addition to the production of cultivated/hybrid meat, the methods for genetically editing genes described herein can be used in other industries. For example, fed-batch processes are used in the pharma/biotech industry to produce recombinant proteins, antibiotics, and yeasts. The methods described herein can also be used in continuous or batch processes.

The above summary is not intended to describe each illustrated embodiment or every implementation of the subject matter hereof. The figures and the detailed description that follow more particularly exemplify various embodiments.

### Brief Description of the Drawings

This disclosure may be more completely understood in consideration of the following description of various embodiments in connection with the accompanying figures, in which:
Figure 1 are tables showing the CRISPR editing next generation sequencing (NGS) for LDHA and PDK1 for the C1F-P2C3 pool according to an embodiment.
Figure 2 are the NGS results for C1F-P4 (clone 6) according to an embodiment.
Figure 3 is a graph showing viable cell density (x10⁶ cells/ml) as a function of days in suspension according to an embodiment.
Figure 4 is a graph showing population double time (hours) as a function of number of passages according to an embodiment.
Figure 5a is a graph showing viable cell density (x 10⁶ cells/ml) as a function of time in culture (days) according to an embodiment.
Figure 5b is a graph showing population doubling time (hours) as a function of passage number according to an embodiment.
Figure 5c is a graph showing the ratio of the change in lactate/glucose concentration as a function of time in culture (days) according to an embodiment.
Figure 6 is a graph showing the cumulative population doubling level (PDL) of C1F-P4 cells from adherent vial thawing and expansion through suspension adaptation and suspension cultivation as a function of time in culture (days) according to an embodiment.
Figure 7a is table showing the results of the sequence analysis of C1F-P4-LDHA according to an embodiment.
Figure 7b is table showing the results of the sequence analysis of C1F-P4-PDK1 according to an embodiment.
Figure 8a is a graph showing viable cell density (VCD) as a function of time (days) for C1F-P2L2 and C1F-P4 cell lines according to an embodiment.
Figure 8b is a graph showing the cumulative population doubling time (PDT) as a function of time (days) for C1F-P2L2 and C1F-P4 cell lines according to an embodiment.
Figure 8c is a graph of lactate concentration (g/L) as a function of time (days) according to an embodiment.
Figure 8d is a graph showing cumulative specific lactate production rate (mg/Mc/day) as a function of time (days) according to an embodiment.
Figure 9a is a graph showing viable cell density (VCD) as a function of time (days) for C1F-P2L2 and C1F-P4 cell lines in suspension cultivation according to an embodiment.
Figure 9b is a graph showing the cumulative population doubling time (PDT) as a function of time (days) for C1F-P2L2 and C1F-P4 cell lines in suspension cultivation according to an embodiment.
Figure 9c is a graph of lactate concentration (g/L) as a function of time (days) in suspension cultivation according to an embodiment.
Figure 9d is a graph showing cumulative specific lactate production rate (mg/Mc/day) as a function of time (days) in suspension cultivation according to an embodiment.
Figure 10a is a graph of the oxygen consumption rate (OCR) (pmol/min) as a function of time (minutes) according to an embodiment.
Figure 10b is a graph of oxygen consumption rate (OCR) (pmol/min) as a function of time (minutes) according to an embodiment.
Figure 11a is graph showing viable cell density (million cells/mL) for C1F-P2L2 and C1F-P4 as a function of time (days) according to an embodiment.
Figure 11b is a graph showing lactate concentration (g/L) as a function of time (days) according to an embodiment.
Figure 11c is a graph showing specific lactate protection (Lac/e3 cells) as a function of time (days) according to an embodiment.
Figure 12a is a graph showing viable cell density (VCD, x10⁶ cell/ml) as a function of time (hours) for C1F-P2L2 and C1F-P4 without (circle and diamond solid line) and with (circle and diamond dashed line) soy lysate according to an embodiment.
Figure 12b is a graph showing the cumulative population doubling time (PDT) as a function of time (days) for C1F-P2L2 and C1F-P4 without and with soy lysate according to an embodiment.
Figure 12c is a graph showing cumulative specific lactate production rate (mg/Mc/day) as a function of time (days) for C1F-P2L2 and C1F-P4 without and with soy lysate according to an embodiment.
Figure 13a is a graph showing the viable cell density (VCD) e6 cells/mL as a function of run time (hours) according to an embodiment.
Figure 13b is a graph showing lactate concentration (g/L) as a function of run time (hours) according to an embodiment.
Figure 13c is a graph showing N-stage specific lactate production rate (g/L/e6 cells/hr) as a function of run time (hour) according to an embodiment.

While various embodiments are amenable to various modifications and alternative forms, specifics thereof have been shown by way of example in the drawings and will be described in detail. It should be understood, however, that the intention is not to limit the disclosure or claims to the particular embodiments described. On the contrary, the intention is to cover all modifications, equivalents, and alternatives falling within the spirit and scope of the subject matter as defined by the claims.

### Detailed Description

### I. Definitions

"Construct", as used herein, refers to recombinant polynucleotides including, without limitation, DNA and RNA, which may be single-stranded or double-stranded and may represent the sense or the antisense strand. Recombinant polynucleotides are polynucleotides formed by laboratory methods that include polynucleotide sequences derived from at least two different natural sources or they may be synthetic. Constructs thus may include new modifications to endogenous genes introduced by, for example, genome editing technologies. Constructs may also include recombinant polynucleotides created using, for example, recombinant DNA methodologies. The constructs provided herein may be prepared by methods available to those of skill in the art, including recombinant techniques. Generally, the nomenclature used herein and the laboratory procedures utilized herein include molecular, biochemical, and recombinant DNA techniques that are commonly employed in the art. Standard techniques available to those skilled in the art may be used for cloning, DNA and RNA isolation, amplification, and purification. The constructs provided herein may include a promoter operably linked to any one of the polynucleotides described herein. The promoter may be a heterologous promoter or an endogenous promoter associated with the biosynthetic pathways described herein.

"crRNA", as used herein, refers to RNAs which combine with Cas proteins to form an effector complex which recognizes the target sequence in the invasive nucleic acid by base pairing to the complementary strand and induces sequence-specific cleavage, thereby preventing proliferation and propagation of foreign genetic elements.

"Cultivated meat" and "cultured meat" are used interchangeably and refers to a form of cellular agriculture where meat is produced by culturing animal cells *in vitro.* "Hybrid meat" as used herein refers to cultivated meat + a plant-based protein, such as a pulse protein.

"Genome edited," "genetically modified," and "genetically engineered," as used herein, refers to a modification to one or more of a cell's genome compared to a non-genome edited cell of the same type (e.g., wild-type). "Genome edited," "genetically modified," and "genetically engineered" can refer to a prokaryotic or eukaryotic cell modified to incorporate or insert an exogenous material (e.g., polynucleotide), regardless of the method used for insertion/incorporation. "Genome edited," "genetically modified," and "genetically engineered" also refers to prokaryotic or eukaryotic cell that has been modified to contain a non-naturally occurring nucleic acid molecule that has been created or modified by the hand of man (e.g., using recombinant DNA technology) or is derived from such a molecule (e.g., by transcription, translation, etc.). Genetically editing or modifying a cell can refer to modifying cellular nucleic acid within a cell, including genetic modifications to endogenous and/or exogenous nucleic acids within the cell. Genetic modifications can include deletions, insertions, integrations of exogenous DNA, gene correction and/or gene mutation. For example, gene editing can be performed using a nuclease (e.g., a natural-existing nuclease or an artificially engineered nuclease) or a transposase. Other methods of making genetic modifications suitable for use according to the methods provided herein include but are not limited to somatic cell nuclear transfer (SCNT) and introduction of a transgene. Procedures for obtaining recombinant or genetically modified cells are generally known in the art, and are described in Sambrook et al., Molecular Cloning, A Laboratory Manual, Cold Spring Harbor Press, Cold Spring Harbor, N.Y. (1989), incorporated herein by reference.

"Guide RNA," as used herein, refers to a piece of RNA that functions as a guide for RNA-or DNA-targeting enzymes, with which it forms complexes. These enzymes can delete, insert or otherwise alter the targeted RNA or DNA.

"Heterologous promoter," "promoter," "promoter region," or "promoter sequence" refer generally to transcriptional regulatory regions of a gene, which may be found at the 5' or 3' side of the polynucleotides described herein, or within the coding region of the polynucleotides, or within introns in the polynucleotides. Typically, a promoter is a DNA regulatory region capable of binding RNA polymerase in a cell and initiating transcription of a downstream (3' direction) coding sequence. The typical 5' promoter sequence is bounded at its 3' terminus by the transcription initiation site and extends upstream (5' direction) to include the minimum number of bases or elements necessary to initiate transcription at levels detectable above background. Within the promoter sequence is a transcription initiation site (conveniently defined by mapping with nuclease SI), as well as protein binding domains (consensus sequences) responsible for the binding of RNA polymerase. In some embodiments, polynucleotides encoding the biosynthetic pathway enzymes described herein are operably connected to the promoter. As used herein, a polynucleotide is "operably connected" or "operably linked" when it is placed into a functional relationship with a second polynucleotide sequence. For instance, a promoter is operably linked to a polynucleotide if the promoter is connected to the polynucleotide such that it may affect transcription of the polynucleotides. In various embodiments, the polynucleotides may be operably linked to at least 1, at least 2, at least 3, at least 4, at least 5, or at least 10 promoters.

Heterologous promoters useful in the practice of the present invention include, but are not limited to, constitutive, inducible, temporally-regulated, developmentally regulated, chemically regulated, tissue-preferred and tissue-specific promoters. The heterologous promoter may be a plant, animal, bacterial, fungal, or synthetic promoter. Suitable promoters for expression in plants include, without limitation, the 35S promoter of the cauliflower mosaic virus, ubiquitin, tCUP cryptic constitutive promoter, the Rsyn7 promoter, pathogen-inducible promoters, the maize In2-2 promoter, the tobacco PR- la promoter, glucocorticoid-inducible promoters, estrogen-inducible promoters and tetracycline-inducible and tetracycline-repressible promoters. Other promoters include the T3, T7 and SP6 promoter sequences, which are often used for in vitro transcription of RNA. In mammalian cells, typical promoters include, without limitation, promoters for Rous sarcoma virus (RSV), human immunodeficiency virus (HIV-1), cytomegalovirus (CMV), Dox-inducible promoter (e.g., Tet Response Element (TRE)), ubiquitin C (Ubc), CMV early enhancer/chicken beta actin (CAG), human beta actin, phosphoglycerate kinase 1 (PGK1), SV40 virus, and the like as well as the translational elongation factor EF-la promoter or ubiquitin promoter. In insect cells, typical promoters include, without limitation, upstream activating sequence (UAS), actin 5c (Ac5), and polyhedrin. In fish cells, typical promoter include, without limitation, Xenopus laevis elongation factor la promoter (XlEeflal) and ocean pout antifreeze protein promoter (OP5a) Those of skill in the art are familiar with a wide variety of additional promoters for use in various cell types. In some embodiments, the heterologous promoter includes a plant promoter, either endogenous to the plant host or heterologous.

"Introducing," as used herein, refers to a process by which exogenous polynucleotides (e.g., DNA or RNA) are introduced into a recipient cell. Methods of introducing polynucleotides into a cell are known in the art and may include, without limitation, microinjection, transformation, and transfection methods. Transformation or transfection may occur under natural or artificial conditions according to various methods well known in the art and may rely on any known method for the insertion of foreign nucleic acid sequences into a host cell. The method for transformation or transfection is selected based on the type of host cell being transformed.

The term "knock-out" refers to an engineered cell, or a method to produce an engineered cell, in which a gene that is naturally present in the host cell is (endogenous gene) is deleted or altered in a manner to prevent or reduce expression of the endogenous gene.

"Metabolite," as used herein, refers to a chemical compound that is an intermediate, end-product, or by-product of primary or secondary metabolism in a cell through one or more biosynthetic pathways. Cellular metabolites can have various functions in fuel, structure, signaling, stimulatory and inhibitory effects on proteins and enzymes, enzymatic co-factors or co-substrates, defense, and interactions with other organisms or cells (e.g., pigments, odorants, pheromones, quorum sensing, etc.).

"Modified cell line" or "engineered cell line" are used interchangeably and refer to cells lines that have been modified or manipulated to decrease or eliminate the production of one or more by-products that adversely affect cell proliferation in the production of cultivated meat.

"Polynucleotide", "polynucleotide sequence", "nucleic acid", and "nucleic acid sequence" refer to a nucleotide, oligonucleotide, polynucleotide (which terms may be used interchangeably), or any fragment thereof. These phrases also refer to DNA or RNA of natural or synthetic origin (which may be single-stranded or double-stranded and may represent the sense or the antisense strand). The polynucleotides may be cDNA or genomic DNA.

Polynucleotides homologous to the polynucleotides described herein are also provided. Those of skill in the art understand the degeneracy of the genetic code and that a variety of polynucleotides can encode the same polypeptide. In some embodiments, the polynucleotides (i.e., polynucleotides encoding the biosynthetic pathway enzymes to synthesize a metabolite of interest) may be codon-optimized for expression in a particular cell including, without limitation, a mammalian cell, a plant cell, bacterial cell, or fungal cell. In some embodiments, the polynucleotide is codon-optimized for genus- or species-specific expression, for example, expression in a bovine cell. While examples of particular polynucleotide sequences are disclosed herein, any polynucleotide sequences may be used which encode a desired form of the polypeptides described herein. Thus, non-naturally occurring sequences may be used. These may be desirable, for example, to enhance expression in heterologous expression systems of polypeptides or proteins. Computer programs for generating degenerate coding sequences are available and can be used for this purpose. Pencil, paper, the genetic code, and a human hand can also be used to generate degenerate coding sequences.

"Polypeptide", "protein", and "peptide", as used herein, are used interchangeably and refer to the collective primary, secondary, tertiary, quaternary amino acid sequence and structure necessary to give the recited macromolecule it function and properties. As used herein, "enzyme" or "biosynthetic pathway enzyme" are used interchangeably and refer to a protein that catalyzes a chemical reaction. The recitation of any particular enzyme either independently or as part of a biosynthetic pathway is understood to include the necessary co-factors, co-enzymes, and metals necessary for the enzyme to properly function.

"Selectable marker", as used herein, refers to a genetic element that allows identification and selection of a cell that contains the construct or vector with said genetic element by expression of said genetic element in the cell. In some embodiments, the selectable marker is a polynucleotide encoding fluorescent protein such that cells expressing the fluorescent protein can be identified visually or by a suitable cell sorting method (e.g., fluorescent activated cell sorting (FACS)). In some embodiments, the selectable marker is a polynucleotide that confer antibiotic resistance to a cell expressing the selectable marker (e.g., puromycin, penicillin, streptomycin, or hygromycin resistance genes). Cells transduced with the vector incorporating the selectable marker can be exposed to a selection chemical specific to the selectable marker to select for cells containing the vector. The selectable marker confer resistance to the selection chemical such that cell containing the vector and the selectable marker survive whereas cells that do not contain the vector and the selectable marker are killed.

"sgRNA", as used herein, refers to "single guide RNA". sgRNA is a single RNA molecule that contains both the custom-designed short crRNA sequence fused to the scaffold tracrRNA sequence.

Trans-activating CRISPR (tracr) RNA, as used herein, refers to a distinct RNA species that interacts with the CRISPR (cr) RNA to form the dual guide (g) RNA in type II and subtype V-B CRISPR-Cas systems. The tracrRNA-crRNA interaction is needed for pre-crRNA processing as well as target recognition and cleavage.

"Vector", as used herein, refers to a polynucleotide capable of transporting another polynucleotide to which it has been linked. In some embodiments, the vector may be a "plasmid," which refers to a circular double-stranded DNA loop into which additional DNA segments may be ligated. Certain vectors are capable of autonomous replication in a host cell into which they are introduced (e.g., bacterial vectors having a bacterial origin of replication and episomal mammalian vectors). Other vectors can be integrated into the genome of a host cell upon introduction into the host cell, and thereby are replicated along with the host genome, such as some viral vectors or transposons. By way of example, appropriate vectors for the compositions and methods of this disclosure include episomal vectors, viral vectors (e.g., retrovirus, adenovirus, baculovirus), plasmids, RNA vectors, or linear or circular DNA or RNA molecules which may comprise or consist of a chromosomal, non-chromosomal, semi-synthetic, or synthetic nucleic acid. Large numbers of suitable vectors are known to those of skill in the art and commercially available. In some embodiments, vectors are episomal vectors, which are capable of autonomous replication due to the presence of an origin of replication. Plant mini chromosomes are also included as vectors. In some embodiments, the vector is a multicistrinic vector including one or more internal ribosomal entry sites (IRES) and/or one or more 2A peptide sequences that allow for co-expression of multiple polynucleotides from a single construct or vector. Vectors may carry genetic elements, such as those that confer resistance to certain drugs or chemicals.

Recitation of ranges of values herein are merely intended to serve as a shorthand method of referring individually to each separate value falling within the range, unless otherwise indicated herein, and each separate value is incorporated into the specification as if it were individually recited herein.

Use of the term "about" is intended to describe values either above or below the stated value in a range of approx. +/- 10%; in other forms the values may range in value either above or below the stated value in a range of approx. +/- 5%; in other forms the values may range in value either above or below the stated value in a range of approx. +/- 2%; in other forms the values may range in value either above or below the stated value in a range of approx. +/- 1%. The preceding ranges are intended to be made clear by context, and no further limitation is implied.

Every compound disclosed herein is intended to be and should be considered to be specifically disclosed herein. Further, every subgroup that can be identified within this disclosure is intended to be and should be considered to be specifically disclosed herein. As a result, it is specifically contemplated that any compound, or subgroup of compounds can be either specifically included for or excluded from use or included in or excluded from a list of compounds.

Disclosed are the components to be used to prepare the disclosed compositions as well as the compositions themselves to be used within the methods disclosed herein. These and other materials are disclosed herein, and it is understood that when combinations, subsets, interactions, groups, etc. of these materials are disclosed that while specific reference of each various individual and collective combinations and permutation of these compounds may not be explicitly disclosed, each is specifically contemplated and described herein. For example, if a particular polypeptide is disclosed and discussed and a number of modifications that can be made to a number of polypeptides are discussed, specifically contemplated is each and every combination and permutation of polypeptides and the modifications that are possible unless specifically indicated to the contrary. Thus, if a class of molecules A, B, and C are disclosed as well as a class of molecules D, E, and F and an example of a combination molecule, A-D is disclosed, then even if each is not individually recited each is individually and collectively contemplated meaning combinations, A-E, A-F, B-D, B-E, B-F, C-D, C-E, and C-F are considered disclosed. Likewise, any subset or combination of these is also disclosed. Thus, for example, the sub-group of A-E, B-F, and C-E would be considered disclosed. This concept applies to all aspects of this application including, but not limited to, steps in methods of making and using the disclosed compositions. Thus, if there are a variety of additional steps that can be performed it is understood that each of these additional steps can be performed with any specific embodiment or combination of embodiments of the disclosed methods.

### II. Genetic Engineered Cell Lines

### A. Cell Line Source

In some embodiments, the cell lines are obtained from an animal, particularly an animal typically consumed by humans. Animals that are commonly captive bred and consumed by humans include vertebrates, invertebrates, and insects. Examples of vertebrates include chicken, duck, turkey, goose, quail, pigeon ostrich, cow, pig, lamb, goat horse, rabbit and fish, Examples of invertebrates include crustaceans such as shrimp, crab, lobster, crayfish octopus, squid, oyster, mussel, and snail.

As used herein, a bird of the genus *Gallus* is an animal that is farmed for human consumption. In some embodiments, the avian cells are selected from, but not limited to, chicken, pheasant, goose, swan, pigeon, turkey, and duck. In some embodiments, the cells are *Gallus* cells. In some embodiments, the cells are selected from, but not limited to, domesticated chicken (*Gallus gallus*). In some embodiments, the avian cell line contains/is an avian fibroblast cell line. In some embodiments, the avian fibroblast cell line contains/is primary avian fibroblast cells. In some embodiments, the avian fibroblast cell line contains/is secondary avian fibroblast cells.

As used herein, a bovine of the *Bos* genus is an animal that is farmed for human consumption. Species of *Bos include B. buiaensis, B. frontails, B. grunniens, B. javanicus, B. savueli, and B. taurus.* In some embodiments, the cells are *Bos taurus* cells. In some embodiments, the cells are selected from, but not limited to *Bos taurus* breeds: Angus, Charolais, Hereford, Simmental, Longhorn, Gelbvieh, Holstein, Limousin, Highlands, and Wagyu. In some embodiments, the cells contains/are primary *Bos taurus* cells. In some embodiments, the cells contains/are secondary *Bos taurus* cells.

As used herein, an animal of the genus *Sus*, is commonly referred to as a pig. The domestic pig is *Sus scrofa domesticus.*

One or more cell lines, for example one or more of the cell lines discussed above, which have been genetically modified or engineered to reduce or eliminate the production/synthesis of by-products (e.g., metabolic waste products) which adversely affect cell proliferation, density, and/or viability are described herein.

### B. Reduction of molecules that adversely affect cell viability and/or proliferation

Cellular metabolism is an important driver of cellular activities; it can adjust to meet the needs of cellular activity. For example, in proliferating cells, aerobic glycolytic metabolism increases, and the intermediate products of glycolytic metabolism are used as building blocks for synthesis of macromolecules needed for cell proliferation. However, the metabolites produced by cellular metabolism also regulate cell activity. Studies have demonstrated that intracellular metabolites are mostly used as donors to modify biological macromolecules, thus regulating cell activity. This is seen with acetyl-CoA, which is not only a metabolite of glucose and fatty acid metabolism, but also a donor of acetyl group for protein acetylation. Similarly, acyl groups for succinylation, crotonylation, lactylation, and other protein acylation modifications are produced by metabolic processes such as the tricarboxylic acid cycle, amino acid metabolism, fatty acid β oxidation, glycolysis, and other metabolic processes. Methyl donors modified with DNA, RNA, and protein methylation are provided by one-carbon metabolism.

Lactate is abundant in rapidly dividing cells owing to the requirement for elevated glucose catabolism to support proliferation. However, it has been reported that lactate, the terminal product of anerobic glycolytic metabolism, binds and inhibits Sentrin/SUMO-specific protease 1 (SENP1). As a consequence of the reduced SENP1 activity, SUMO2/3-modification of anaphase-promoting complex 4 (APC4) is accumulated, promoting degradation of APC/C complexes, and mitosis exit.

In one embodiment, the target by-product or metabolic waste product is lactate. In some embodiments, the one or more genes involved in lactate production that are modified are lactate dehydrogenase A (LDHA), pyruvate dehydrogenase kinase 1 (PDK1) and combinations thereof.

LDHA encodes the A subunit of lactate dehydrogenase enzyme which catalyzes the reversible conversion of pyruvate to lactate with the concomitant oxidation of NADH to NAD in the final step of anaerobic glycolysis. The protein is found predominantly in muscle tissue and belongs to the lactate dehydrogenase family.

PDK1 regulates the catalytic activity of pyruvate decarboxylation oxidation via the mitochondrial pyruvate dehydrogenase complex, and it further links glycolysis with the tricarboxylic acid cycle and ATP generation.

In some embodiments, LDHA and PDK1 are modified or engineered. In some embodiments, LDHA and PDK1 are inactivated or "knocked out".

The target genes, such as LDHA and/or PDK1, can be modified or engineered (e.g., inactivated or "knocked out") using gene editing techniques known in the art. Suitable gene editing tools and techniques include, but are not limited to, zinc-finger nucleases (ZFNs), transcription activator-like effector nucleases (TALENs), meganucleases, and clustered regularly interspaced short palindromic repeats, also known as CRISPR/Cas9.

In some embodiments, knock outs, such as LDHA, PDK1, or both (double) knockouts, are prepared using CRISPR/Cas9. In some embodiments, multiple signal RNAs (sgRNAs) were designed for each target gene. In some embodiments, transfection of the gene was initiated to generate a "knock-out" ("KO") cell pool. In some embodiments, PDK1 KO cell pools were generated. In some embodiments, the KO efficiency of PDK1 is at least 1%, 2%, 3%, 4%, 5%, 6%, 7%, 8%, 9%, 10%, 15%, 20%, 25%, 30%, 35%, 40%, 45%, 50% or higher. In some embodiments, the PDK1 cell pool with the highest KO efficiency was selected and transfected with LDHA gRNAs on the same pool to generate a double KO pool. In some embodiments, the KO efficiency of LDHA is at least 1%, 2%, 3%, 4%, 5%, 6%, 7%, 8%, 9%, 10%, 15%, 20%, 25%, 30%, 35%, 40%, 45%, 50% or higher. In some embodiments, a cell line with a particular knockout efficiency (e.g., 1%-5%) is selected for single cell cloning.

In some embodiments, the modified cell line exhibits improved cell performance (e.g., proliferation, viable cell density, population doubling time, etc.) compared to the unmodified cell line. In some embodiments, proliferation is measured by any method known to one skilled in the art. In some embodiments, proliferation is measured through direct cell counts. In certain embodiments, proliferation is measured by a haemocytometer. In some embodiments, proliferation is measured by automated cell imaging. In certain embodiments, proliferation is measured by a Coulter counter. In some embodiments, proliferation is measured by viability stains. In certain embodiments, the stains used contain, include, or is trypan blue. In some embodiments, proliferation is measured by the total DNA. In some embodiments, proliferation is measured by BrdU labelling. In some embodiments, proliferation is measured by metabolic measurements. In certain embodiments, proliferation is measured by using tetrazolium salts. In certain embodiments, proliferation is measured by ATP-coupled luminescence.

In some embodiments, the modified cell line shows at least a 20%, 25%, 30%, 35%, 40%, 45%, 50%, 55%, or greater improvement in viable cell density (VCD) at day 4 compared to the unmodified cell line when cultured by plate suspension at an initial density of 0.2E+06 cells/well. In some embodiments, the modified cell line exhibits an improved population doubling time (PDT) compared to the unmodified cell line. In some embodiments, the modified cell line exhibits a 10%, 12%, 15%, 17%, 20%, 25% or greater reduction in PDT compared to the unmodified cell line when cultured by plate suspension at an initial density of 0.2E+06 cells/well. In some embodiments, the modified cell line exhibits the improved VCD and PDT described above.

In some embodiments, the modified cell line reached a VCD of at least 15 million cells/mL after three continuous passages in a bioreactor.

In some embodiments, the modified cells had a wet cell weight of at least 10 g/L, 15 g/L, 20 g/L, 25 g/L, 30 g/L, or 35 g/L after three continuous passages in a bioreactor.

Without being bound by any particular theory, the data in the Examples supports the idea that the modified cells are shifting their metabolism (or balancing it more efficiently) from glycolysis to oxidative phosphorylation. For example, the improvements in VCD and PDT described above are likely due not only to lactate reduction but potentially a more efficient use of oxidative phosphorylation. Therefore, in some embodiments, the modified cell lines described herein exhibit improvements in VCD and/or PDT compared to unmodified cells due to a shift, balancing, or selection of one or more metabolic pathways as a result of the genetic modifications.

The disclosed modified cells can be diploid cells with two copies (e.g. alleles) of genes, e.g., the preferred targets of PDK1 and LDHA. The two alleles in a diploid cell can be the same or different. In some embodiments, only one allele of target gene is modified (e.g., knocked out or inactivated). In other embodiments, both alleles of a target gene are modified (e.g., knocked out or inactivated).

In some embodiments, the modified cell exhibiting the above improvements is a single KO, such as PDK1 KO or LDHA KO. In other embodiments, the modified cell line is a double KO, such as PDK1 and LDHAKO.

### C. Cell Culture Media

In some embodiments, the modified cell lines described herein are cultured in standard media. In some embodiments, the modified cell lines described herein are adapted to media, which is free of direct growth factors, indirect growth factors, animal serum, and/or animal-derived components. Such adaption procedures and media compositions are described in pending PCT Application Serial No. PCT/US2023/069582, which is incorporated herein by reference.

In other embodiments, one or more of the maintenance, proliferation, differentiation, lipid accumulation, lipid content, proneness to purification and/or harvest efficiency, growth rates, cell densities, cell weight, resistance to contamination, expression of endogenous genes and/or protein secretion, shear sensitivity, flavor, texture, color, odor, aroma, gustatory quality, nutritional quality, minimized growth-inhibitory byproduct secretion, and/or minimized media requirements, of cultivated animal cells, in any culture conditions, are improved by the use of one or more of growth factors, proteins, peptides, fatty acids, elements, small molecules, plant hydrolysates, directed evolution, genetic engineering, media composition, bioreactor design, and/or scaffold design.

In certain embodiments, the fatty acids include or is stearidonic acid (SDA). In certain embodiments, the fatty acids include or is linoleic acid.

In certain embodiments, the growth factor includes or is insulin or insulin like growth factor. In certain embodiments, the growth factor includes or is fibroblast growth factor or the like. In certain embodiments, the growth factor includes or is epidermal growth factor or the like. In certain embodiments, the protein includes or is transferrin.

In certain embodiments, the element includes or is selenium.

In certain embodiments, a small molecule includes or is ethanolamine. In some embodiments, the amount of ethanolamine used in the cultivations is between 0.05-10 mg/L, 0.05-10 mg/L, 0.1-10 mg/L, 0.1-9.5 mg/L, 0.1-9 mg/L, 0.1-8.5 mg/L, 0.1-8.0 mg/L, 0.1-7.5 mg/L, 0.1-7.0 mg/L, 0.1-6.5 mg/L, 0.1-6.0 mg/L, 0.1-5.5 mg/L, 0.1-5.0 mg/L, 0.1-4.5 mg/L, 0.1-4.0 mg/L, 0.1-3.5 mg/L, 0.1-3.0 mg/L, 0.1-2.5 mg/L, 0.1-2.0 mg/L, 0.1-1.5 mg/L, and 0.1-1.0 mg/L.

In certain embodiments, the media can be supplemented with plant hydrolysates. In certain embodiments, the hydrolysates is selected from one or more yeast extract, wheat peptone, rice peptone, phytone peptone, yeastolate, pea peptone, soy peptone, pea peptone, potato peptone, mung bean protein hydrolysate, or sheftone. In some embodiments, the amount of hydrolysate is between 0.1 g/L to 5 g/L, between 0.1 g/L to 4.5 g/L, between 0.1 g/L to 4 g/L, between 0.1 g/L to 3.5 g/L, between 0.1 g/L to 3 g/L, between 0.1 g/L to 2.5 g/L, between 0.1 g/L to 2 g/L, between 0.1 g/L to 1.5 g/L, between 0.1 g/L to 1 g/L, or between 0.1 g/L to 0.5 g/L.

### III. Food Products

Food products containing, or made from, the cultivated animal cells described herein are also disclosed. In some embodiments, there are provided methods of producing a food product containing one or more of the cell types described above cultured in the growth medium described above. In some embodiments, the methods include culturing a population of cells in vitro in a growth medium capable of maintaining the cells, recovering cells, and formulating the recovered cells into an edible food product. In some embodiments, the cells include fibroblasts, muscle cells, myosatellite cells, myoblasts, fat cells, pre-adipocytes, adipocytes, epithelial cells, or combinations thereof.

### A. Cell Culture

In some embodiments, the cultivated animal cells are grown in a suspension culture system. In some embodiments, the cells are grown in a batch, fed-batch, semi continuous (fill and draw) or perfusion culture system or some combination thereof. When grown in suspension culture, the suspension culture can be performed in a vessel (cultivation tank, bioreactor)) of a desired size. The vessel is a size that is suitable for growth of cells without unacceptable rupture of the cells. In some embodiments, the suspension culture system can be performed in vessel that is at least 25 liters (L), 50L, 100L, 200L, 250L, 350L, 500L, 1000L, 2,500L, 5,000L, 10,000L, 25,000L, 50,000L, 100,000L, 200,000L, 250,000L, or 500,000L. For smaller suspension cultures, the cultivation of the cells can be performed in a flask that is least 125 mL, 250 mL, 500 mL, 1 L, 1.5 L, 2 L, 2.5 L, 3 L, 5 L, 10L, or larger.

In some embodiments, the cell density of the suspension culture is between 0.25x 10⁶ cells.ml, 0.5×10⁶ cells/ml and 1.0× 10⁶ cells/ml, between 1.0× 10⁶ cells/ml and 2.0× 10⁶ cells/ml, between 2.0× 10⁶ cells/ml and 3.0× 10⁶ cells/ml, between 3.0× 10⁶ cells/ml and 4.0× 10⁶ cells/ml, between 4.0× 10⁶ cells/ml and 5.0× 10⁶ cells/ml, between 5.0× 10⁶ cells/ml and 6.0× 10⁶ cells/ml, between 6.0× 10⁶ cells/ml and 7.0× 10⁶ cells/ml, between 7.0× 10⁶ cells/ml and 8.0× 10⁶ cells/ml, between 8.0× 10⁶ cells/ml and 9.0× 10⁶ cells/ml, between 9.0× 10⁶ cells/ml and 10× 10⁶ cells/ml, between 10× 10⁶ cells/ml and 15.0× 10⁶ cells/ml, between 15× 10⁶ cells/ml and 20× 10⁶ cells/ml, between 20× 10⁶ cells/ml and 25×10⁶ cells/ml, between 25× 10⁶ cells/ml and 30× 10⁶ cells/ml, between 30× 10⁶ cells/ml and 35× 10⁶ cells/ml, between 35× 10⁶ cells/ml and 40× 10⁶ cells/ml, between 40× 10⁶ cells/ml and 45 ×10⁶ cells/ml, between 45× 10⁶ cells/ml and 50× 10⁶ cells/ml, between 50× 10⁶ cells/ml and 55× 10⁶ cells/ml, between 55× 10⁶cells/ml and 60× 10⁶ cells/ml, between 60× 10⁶ cells/ml and 65× 10⁶ cells/ml, between 70× 10⁶ cells/ml and 75× 10⁶ cells/ml, between 75× 10⁶ cells/ml and 80× 10⁶ cells/ml, between 85× 10⁶ cells/ml and 90× 10⁶ cells/ml, between 90× 10⁶ cells/ml and 95× 10⁶ cells/ml, between 95× 10⁶ cells/ml and 100× 10⁶ cells/ml, between 100× 10⁶ cells/ml and 125× 10⁶ cells/ml, or between 125× 10⁶ cells/ml and 150× 10⁶ cells/ml.

In some embodiments, the cultivated animal cells are grown while embedded in scaffolds or attached to scaffolding materials. In some embodiments, the cultivated animal cells are differentiated or proliferated in a bioreactor and/or on a scaffold.

In some embodiments, the scaffold contains at least one or more of a microcarrier, an organoid and/or vascularized culture, self-assembling co-culture, a monolayer, hydrogel scaffold, decellularized animal product, such as decellularized meat, decellularized connective tissue, decellularized skin, decellularized offal, or other decellularized animal byproducts, and/or an edible matrix. In some embodiments, the scaffold contains at least one of plastic and/or glass or other material. In some embodiments, the scaffold contains natural-based (biological) polymers chitin, alginate, chondroitin sulfate, carrageenan, gellan gum, hyaluronic acid, cellulose, collagen, gelatin, and/or elastin.

In some embodiments, the scaffold contains a protein or a polypeptide, or a modified protein or modified polypeptide. The unmodified protein or polypeptide or modified protein or polypeptide includes proteins or polypeptides isolated from plants or other organisms. Exemplary plant protein isolates or plant protein concentrates contain pulse protein, vetch protein, grain protein, nut protein, macroalgal protein, microalgal protein, and other plant proteins. Pulse protein can be obtained from dry beans, lentils, mung beans, favabeans, dry peas, chickpeas, cowpeas, bambara beans, pigeon peas, lupins, vetches, adzuki, common beans, fenugreek, long beans, lima beans, runner beans, or tepary beans, soybeans, or mucuna beans. Vetch protein can be obtained from the genus Vicia. Grain protein can be obtained from wheat, rice, teff, oat, corn, barley, sorghum, rye, millet, triticale, amaranth, buckwheat, quinoa and other grains. Nut protein can be obtained from almond, cashew, pecan, peanut, walnut, macadamia, hazelnut, pistachio, brazil, chestnut, kola nut, sunflower seeds, pumpkin seeds, flax seeds, cacao, pine nut, ginkgo, and other nuts. Proteins obtained from animal source can also be used as scaffolds, including milk proteins, whey, casein, egg protein, and other animal proteins.

In some embodiments, the self-assembling co-cultures contains spheroids and/or aggregates. In some embodiments, the monolayer is with or without an extracellular matrix. In some embodiments, the hydrogel scaffolds contains at least one of hyaluronic acid, alginate and/or polyethylene glycol. In some embodiments, the edible matrix contains decellularized plant tissue. Vegetable and animal protein, both modified and unmodified, can be extruded in an extrusion machine to prepare an extrudate that can be used as a scaffold for adherent cell culture of cultivated animal cells. Cultivated animal cells or cells isolated from the tissue of an animal, for example a cultivated *Gallus Gallus* cells or *Bos taurus* cells as disclosed herein can be processed through an extrusion machine to make an extrudate, which extrudate can be used as a scaffold for cultivation of cells.

The cells can be recovered by any technique apparent to those of skill. In some embodiments the cells are separated from the growth media or are removed from a bioreactor or a scaffold. In certain embodiments, the cultivated animal cells are separated by centrifugation, a mechanical/filter press, filtration, flocculation or coagulation or gravity settling or drying or some combination thereof. In certain embodiments, the filtration method contains tangential flow filtration, vacuum filtration, rotary vacuum filtration and similar methods. In certain embodiments the drying can be accomplished by flash drying, bed drying, tray drying and/or fluidized bed drying and similar methods. In certain embodiments, the cells are separated enzymatically. In certain embodiments, the cells are separated mechanically.

In some embodiments, the population of cultivated animal cells is substantially pure.

In some embodiments, tests are administered at one or more steps of cell culturing to determine whether the cultivated animal cells are substantially pure.

In some embodiments, the cultivated animal cells are tested for the presence or absence of bacteria. In certain embodiments, the types of bacteria tested include, but are not limited to: *Salmonella enteritidis, Staphylococcus aureus, Campylobacter jejunim, Listeria monocytogenes, Fecal streptococcus, Mycoplasma genus, Mycoplasma pulmonis, Coliforms, and Escherichia coli.*

In some embodiments, components of the cell media, such as fetal bovine serum, are tested for the presence or absence of viruses. In certain embodiments, the viruses include, but are not limited to: Bluetongue, Bovine Adenovirus, Bovine Parvovirus, Bovine Respiratory Syncytial Virus, Bovine Viral Diarrhea Virus, Rabies, Reovirus, REV, AEV, ALVA, ALVB, ALVJ, FAV1, FAV3, CAV, ARV, Avian S. pullorum, Avian Mycoplasma Genus, Adeno-associated virus, BK virus, Epstein-Barr virus, Hepatitis A virus, Hepatitis B virus, Hepatitis C virus, Herpes Simplex 1, Herpes Simplex 2 , Herpes virus type 6, Herpes virus type 7, Herpes virus type 8, HIV1, HIV-2, HPV-16, HPV 18, Human cytomegalovirus, Human Foamy virus, Human T-lymphotropic virus, John Cunningham virus, and Parvovirus B19.

In some embodiments, the tests are conducted for the presence or absence of yeast and/or molds.

In some embodiments, the tests are for metal concentrations by mass spectrometry, for example inductively coupled plasma mass spectrometry (ICP-MS). In certain embodiments, metals tested include, but are not limited to, arsenic, lead, mercury, cadmium, and chromium.

In some embodiments, the tests are for hormones produced in the culture. In certain embodiments, the hormones include, but are not limited: to 17β-estradiol, testosterone, progesterone, zeranol, melengesterol acetate, trenbolone acetate, megestrol acetate, chlormadinone acetate, dienestrol, diethylstilbestrol, hexestrol, taleranol, zearalanone, and zeranol.

In some embodiments, the tests are in keeping with the current good manufacturing process as detailed by the United States Food and Drug Administration.

### B. Food Product Production

In certain embodiments provided herein are food compositions or food products containing cultivated animal cells that are cultivated *in vitro.* In some embodiments, the cells are combined with other substances or ingredients (e.g., fat, plant-based protein, etc.) to make a composition that is an edible food product composition. In certain embodiments, the cultivated animal cells are used alone to make a composition that is a food product composition. In certain embodiments, the food product composition is a product that resembles nuggets, tenders, bites, steak, roast, ground meat, hamburger patties, sausage, or feed stock.

In some embodiments, the method for producing a food product includes: (1) conditioning water with a phosphate to prepare conditioned water; (2) hydrating a plant protein isolate or plant protein concentrate, such as a pulse protein isolate or concentrate, with the conditioned water to produce hydrated plant protein; (3) contacting the animal cells with the hydrated plant protein to produce a cell and pulse protein mixture; (4) heating the cell and plant protein mixture in steps, wherein the steps include at least one of: (i) ramping up the temperature of the cell and protein mixture to a temperature between 40-65°C; (ii) maintaining the temperature of the cell and protein mixture at a temperature between 40-65°C for 1 to 30 minutes; (iii) ramping up the temperature of the cell and protein mixture to a temperature between 60-85°C; (iv) cooling the cell and protein mixture to a temperature between -1°C-25°C and (v) admixing the cell and protein mixture with a fat to create a pre-cooking product. In some embodiments, the pre-cooking product can be consumed without further cooking. Alternatively, in some embodiments, the pre-cooking product is cooked to produce the edible food product. Optionally, the pre-cooking product may be stored at room temperature, refrigeration temperatures or frozen.

In some embodiments, the food product is prepared by co-extruding a cell paste and a plant protein isolate/concentrate to form a food product. In some embodiments, the apparatus can be a cooling die that has a body defining a flow path extending from an inlet at an upstream end of the body to an outlet at a downstream end of the body. In some embodiments, the inlet has a first cross-sectional area, and the outlet has a second cross-sectional area that is larger than the first cross-sectional area. Additionally, the flow path can include a transitional region at the upstream end of the body and the flow path includes a forming region extending from the transitional region to the outlet.

In some embodiments, the food composition or food product contains about 1%-100% by weight wet cell paste.

In certain embodiments, the food composition or food product has a wet cell paste content of at least 100%, 90%, 80%, 75%, 70%, 65%, 60%, 50%, 40%, 35%, 25%, 15%, 10%, 5%, 4%, 3%, 2%, or 1% by weight. In certain embodiments, the food composition or food product contains a wet cell paste content of between 1%-5%, 2%-5%, 3%-5%, 5%-10%, 10%-15%, 15%-20%, 20%-25%, 25%-30%, 30%-35%, 35%-40%, 40%-45%, 45%-50%, 50%-55%, 55%-60%, 65%-70%, 70%-75%, 75%-80%, 80%-85%, 85%-90%, or 90%-95%. In certain embodiments, the food composition or food product has a wet cell paste content by weight of between 3%-5%, 5%-10%, 10%-20%, 20%-30%, 30%-40%, 40%- 50%, 60%-70%, 80%-90%, or 90%-100%.

In certain embodiments, the composition has a pulse protein content by weight of at least 85%, 80%, 75%, 70%, 65%, 60%, 55%, 50%, 45%, 40%, 35%, 30%, 25%, 20%, or 15% by weight. In certain embodiments, the food composition or food product has a pulse protein content by weight of between 10%-95%, or any subrange therebetween, e.g., 10%-20%, 20%-30%, 30%-40%, 40%- 50%, 60%-70%, 80%-90%, or 90%-95%.

In certain embodiments, the food composition or food product contains a fat content of at least 50%, 40%, 30%, 25%, 20%, 15%, 10%, 5%, or 1% by weight. In certain embodiments, the food composition or food product has a fat content by weight of between 10%-95%, or any subrange therebetween, e.g., 10%-20%, 20%-30%, 30%-40%, 40%- 50%, 60%-70%, 80%-90%, or 90%-95%.

In certain embodiments, the food composition or food product contains a water content of at least 50%, 40%, 30%, 25%, 20%, 15%, 10% or 5% by weight. In certain embodiments, the food composition or food product has a water content by weight of between 10%-95%, or any subrange therebetween, e.g., 10%-20%, 20%-30%, 30%-40%, 40%- 50%, 60%-70%, 80%-90%, or 90-95%.

In certain embodiments, the food composition or food product contains a dry cell weight content of at least of 1% by weight. In certain embodiments, the food composition or food product has a dry cell weight content of at least of 5% by weight. In certain embodiments, the food composition or food product has a dry cell weight content of at least of 10% by weight. In certain embodiments, the food composition or food product has a dry cell weight content of at least of 15% by weight. In certain embodiments, the food composition or food product has a dry cell weight content of at least of 20% by weight. In certain embodiments, the food composition or food product has a dry cell weight content of at least of 25% by weight. In certain embodiments, the composition or food product has a dry cell weight of at least of 30% by weight. In certain embodiments, the composition or food product has a dry cell weight of at least of 35% by weight. In certain embodiments, the composition or food product has a dry cell weight of at least of 40% by weight. In certain embodiments, the composition or food product has a dry cell weight of at least of 45% by weight. In certain embodiments, the composition or food product has a dry cell weight of at least of 50% by weight. In certain embodiments, the composition or food product has a dry cell weight of at least of 55% by weight. In certain embodiments, the composition or food product has a dry cell weight of at least of 60% by weight. In certain embodiments, the composition or food product has a dry cell weight of at least of 65% by weight. In certain embodiments, the composition or food product has a dry cell weight of at least of 70% by weight. In certain embodiments, the composition or food product has a dry cell weight of at least of 75% by weight. In certain embodiments, the composition or food product has a dry cell weight of at least of 80% by weight. In certain embodiments, the composition or food product has a dry cell weight of at least of 85% by weight. In certain embodiments, the composition or food product has a dry cell weight of at least of 90% by weight. In certain embodiments, the composition or food product has a dry cell weight of at least of 95% by weight. In certain embodiments, the composition or food product has a dry cell weight of at least of 97% by weight. In certain embodiments, the composition or food product has a dry cell weight of at least of 98% by weight. In certain embodiments, the composition or food product has a dry cell weight of at least of 99% by weight. In certain embodiments, the composition or food product has a dry cell weight of at least of 100% by weight. In certain embodiments, the food composition or food product has a dry cell weight content of between 1%-100%, or any subrange therebetween, e.g., 2%-5%, 3%-5%, 5%-10%, 10%-15%, 15%-20%, 20%-25%, 25%-30%, 30%-35%, 35%-40%, 40%-45%, 45%-50%, 50%-55%, 55%-60%, 65%-70%, 70%-75%, 75%-80%, 80%-85%, 85%-90%, or 90%-95%.

In certain embodiments, the food composition or food product contains a pulse protein content of at least 2%, 5%, 10 %, 15%, 20%, 25%, 30%, 35%, 40%, 45%, 50%, 55%, 60%, 65%, 70%, 75%, 80%, 85%, 90% or 95% by weight. In certain embodiments, the food composition or food product contains a pulse protein content of between 2%-95%, or any subrange therebetween, e.g., 2%-5%, 5%-10%, 10%-15%, 15%-20%, 20%-25%, 25%-30%, 30%-35%, 35%-40%, 40%-45%, 45%-50%, 50%-55%, 55%-60%, 65%-70%, 70%-75%, 75%-80%, 80%-85%, 85%-90%, or 90%-95%, In some embodiments, the pulse protein is a mung bean protein. In some embodiments, the pulse protein is a soy protein.

In some embodiments, the composition contains a peptide cross-linking enzyme. Exemplary peptide cross-linking enzymes are selected from the group consisting of transglutaminase, sortase, subtilisin, tyrosinase, laccase, peroxidase, and lysyl oxidase. In certain embodiments, the composition contains a cross-linking enzyme of between 0.0001%-0.025%, 0.0001%-0.020%, 0.0001%-0.0175%, 0.0001%-0.0150%, 0.0001%-0.0125%, 0.0001%-0.01%, 0.0001%-0.0075%, 0.0001%-0.005%, 0.0001%-0.0025%, 0.0001%-0.002%, 0.0001%-0.0015%, 0.0001%-0.001%, 0.0001%-0.00015% by weight. In certain embodiments, the food composition or food product comprises a transglutaminase content between 0.0001%-0.025%, 0.0001%-0.020%, 0.0001%-0.0175%, 0.0001%-0.0150%, 0.0001%-0.0125%, 0.0001%-0.01%, 0.0001%-0.0075%, 0.0001%-0.005%, 0.0001%-0.0025%, 0.0001%-0.002%, 0.0001%-0.0015%, 0.0001%-0.001%, 0.0001%-0.00015% by weight. In some embodiments, the composition contains transglutaminase at a concentration from about 0.0001 to about 0.0125%

In one embodiment, the food composition or food product comprises 0.0001% to 0.0125% transglutaminase, and exhibits reduced or significantly reduced lipoxygenase activity or other enzymes which oxidize lipids, as expressed on a volumetric basis relative to cell paste without the transglutaminase. More preferably, the food composition or food product is essentially free of lipoxygenase or enzymes that can oxidize lipids. In some embodiments, a 5%, 10%, 15%, 20%, 25%, 30%, 35%, 40%, 45%, 50%, 55%, 60%, 65%, 70%, 75%, or 80% reduction in oxidative enzymatic activity relative to a composition is observed. Lipoxygenases catalyze the oxidation of lipids that contribute to the formation of compounds that impart undesirable flavors to compositions.

In some embodiments, the formulation to be extruded includes cultivated animal cells in an amount between 35-95 wt% of the formulation, and a dry mix in an amount between 25-45 wt% of the formulation. The dry mix can include a proteinaceous ingredient, a binding ingredient, an emulsifier, and one or more flavorants.

In some embodiments, there are provided food products contain cells of the genus Bos or genus Gallus, the food product containing a cell paste at a content of at least 1% by weight; a plant protein isolate or plant protein concentrate, the plant protein content at least 5% by weight of the food product; a fat, the fat content at least 5% by weight of the food product; and water, the water content at least 5% by weight of the food product. In some embodiments, the food products contains at least 3% by weight cell paste.

In some embodiments, plant protein isolates or plant protein concentrates are obtained from pulses selected from dry beans, lentils, mung beans, fava beans, dry peas, chickpeas, cowpeas, bambara beans, pigeon peas, lupins, vetches, adzuki, common beans, fenugreek, long beans, lima beans, runner beans, or tepary beans, soybeans, mucuna beans, or combinations thereof. In some embodiments, the pulse protein isolate/concentrate is a soybean isolate/concentrate.

In some embodiments, the pulse protein isolates or plant protein concentrates provided herein are derived from *Vigna angularis, Vicia faba, Cicer arietinum, Lens culinaris, Phaseolus vulgaris, Vigna unguiculata, Vigna subterranea, Cajanus cajan, Lupinus sp., Vetch sp., Trigonella foenum-graecum, Phaseolus lunatus, Phaseolus coccineus, or Phaseolus acutifolius.* In some embodiments, the pulse protein isolates are derived from mung beans. In some embodiments, the mung bean is *Vigna radiata.*

In some embodiments, animal protein isolate and animal protein concentrate are obtained from animals or animal products. Examples of animal protein isolate or animal protein concentrate include collagen, whey, casein, and egg protein.

In some embodiments, plant protein isolates are obtained from wheat, rice, teff, oat, corn, barley, sorghum, rye, millet, triticale, amaranth, buckwheat, quinoa, almond, cashew, pecan, peanut, walnut, macadamia, hazelnut, pistachio, brazil, chestnut, kola nut, sunflower seeds, pumpkin seeds, flax seeds, cacao, pine nut, ginkgo, and other nuts.

### C. Non-Food Uses

In some embodiments, the methods described herein can be used in non-food applications. For examples, fed-batch processes are used in the production of recombinant proteins, anti-biotics, and yeasts. The literature reports up to a 150-fold increase in yield compared to batch processes.

### EXAMPLES

### Example 1. C1F-P4 Clone Generation and Bank Generation

### Generation of C1F-P2C3 pool

Three (3) different sgRNAs were designed for each target and *in vitro* transcription to synthesize guide RNAs was performed. Transfection of PDK1 was initiated to generate PDK1 KO cell pool. The cell pool with highest KO efficiency (PDK1.2, 46%) was selected and subsequently transfected with LDHA gRNAs on the same pool to generate a double KO pool. The Double KO pool transfected with LDHA.3 showed the highest KO efficiency (39%, Table 1), The selected guide RNAs are listed in Table 1 and the KO efficiencies are shown in Table 2.

**Table 1. sgRNA information for C1F-P4 and pool KO efficiency**

| **Target gene** | **Thermo Fisher gRNA ID** | **Guide RNA sequence** | **Pool knockout efficiency** |
|---|---|---|---|
| LDHA | LDHA.3 | CAACCACGCTGATCTTGTTG | 39 |
| PDK1 | PDK1.2 | GGTGTTCTGAGAAGATTGTC | 46 |

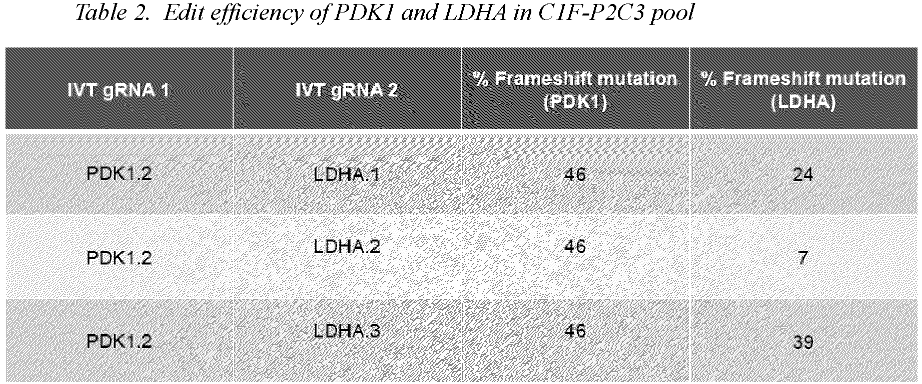

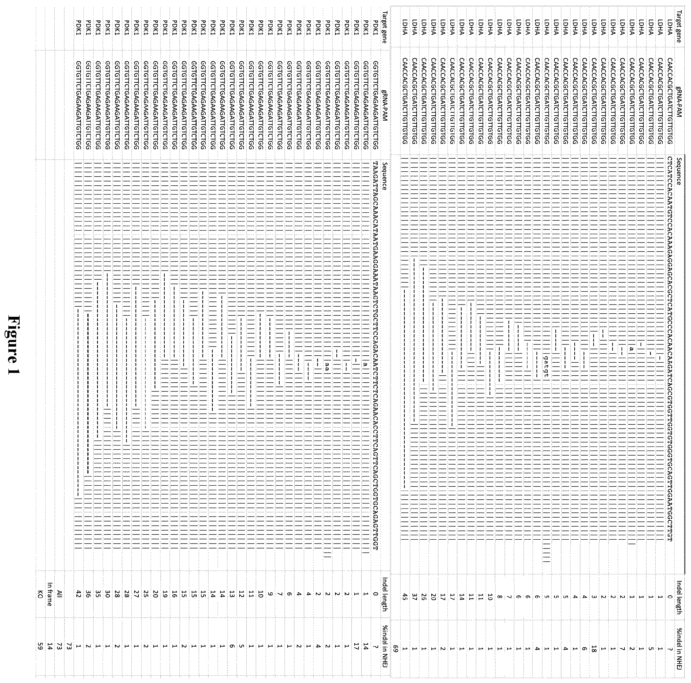

Next Generation Sequencing (NGS) was used to assess the pool edit efficiencies. As shown in Figure 1, NGS results confirmed the pool edit efficiency for both LDHA and PDK1 did not drop after several passages in C1F-P2C3, suggesting (1) the edit population is stable, and (2) double KO do not cause disadvantaged growth compared with the non-edit population.

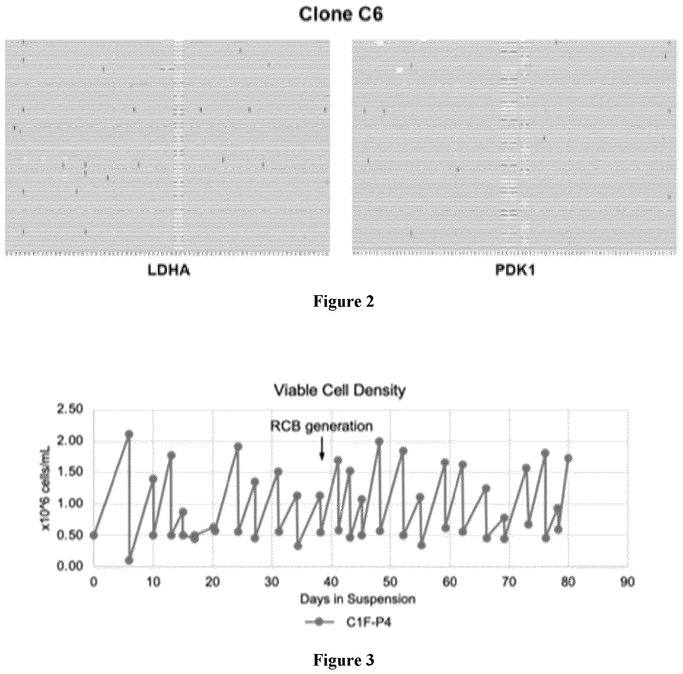

Single cell cloning was performed. Clone C6 was identified as a homozygous KO for both LDHA and PDK1. Clone 6 carries 2 bp deletion for LDHA in both alleles, and 2 bp deletion in one allele and 4 bp deletion in another allele for PDK1. Figure 2 shows the NGS results for clone C6. This cell line is referred to as C1F-P4. The cells were transferred to a liquid nitrogen tank for long term storage.

The cells were thawed and transferred into a 10 mL fresh DMEM/F-12 media with 10% FBS (DF-10). The cells were spun down in 400g for 5 minutes and the supernatant was removed. The cells were resuspended in 5mL DF-10 media. A 0.6mL aliquot was used for ViCell count. The remaining cells were plated into a T25 flask.

To recover cells, the media was refreshed every three to four days to ensure sufficient nutrients for cells and remove floating dead cells. The cells were recovered after two weeks and expanded to T175.

### Suspension and Serum-Free Adaptation

An adherent bank of C1F-P4 cells (p245) was thawed into a T75 flask and subsequently passaged to T175 flasks. The cells were detached with TrypLE and spun down, the media was changed to fresh SFC7, and the cells were resuspended in shake flasks for suspension adaptation.

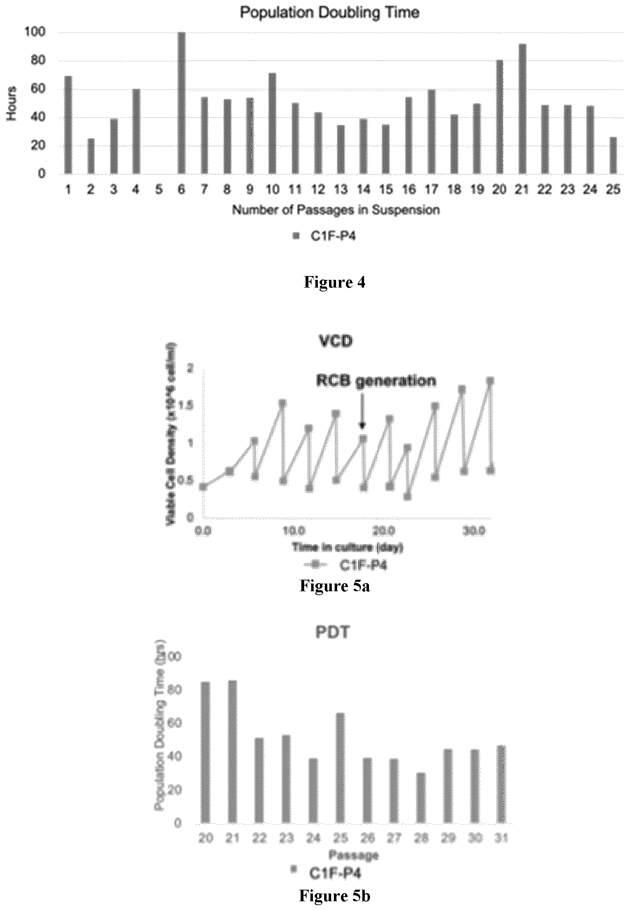

The cells were maintained in suspension culture; once stable growth in serum-free and suspension culture was observed, a suspension RCB was generated to preserve the adapted condition (noted in Figure 3). The growth performance of C1F-P4 during suspension adaptation is shown in Figures 3 and 4.

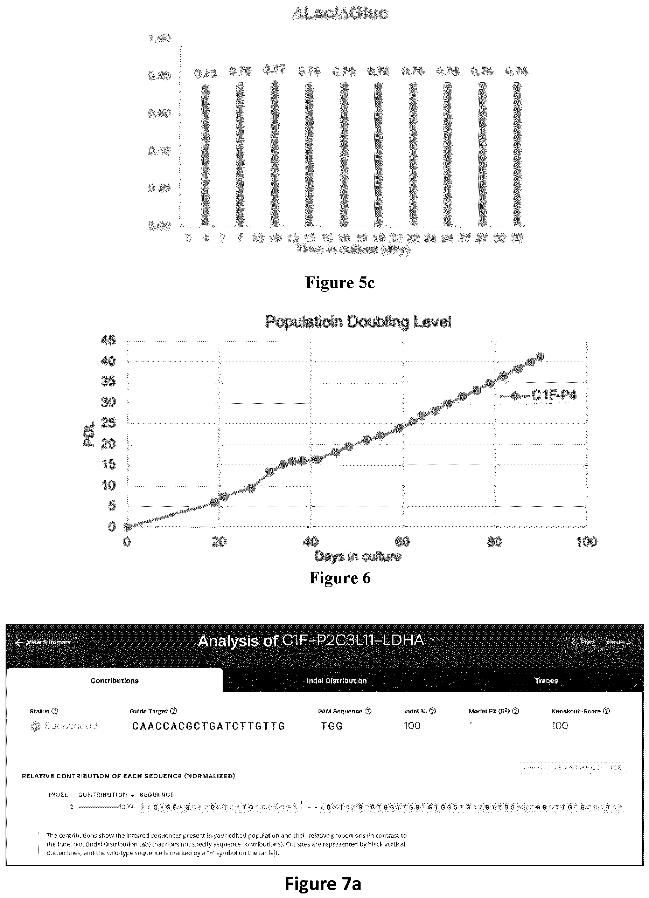

A vial of suspension-adapted C1F-P4 was thawed to validate the performance stability of the RCB and generate a larger RCB. C1F-P4 were thawed and cell performance was stable during scale up as shown in Figures 5a-5c. A larger RCB was generated (noted in Figure 5a). Two vials of this RCB were analyzed for karyotyping, genomic stability, and cell line authentication.

Overall, the suspension RCBs showed stable growth and metabolic performance, suggesting this cell line is suitable for use in yield improvement process development. Cumulative population doubling levels, from the adherent cryovial thaw and expansion, through suspension adaptation and suspension RCB generation, was compiled to demonstrate the stable growth of C1F-P4 cells (Figure 6).

### Validation of CRISPR Knockout in C1F-P4

C1F-P4 is a double KO of LDHA and PDK1 in C1F-P2 cells and showed significant improvement in cell growth and lactate production. To confirm the identify of this cell line, various approaches were used to validate the clone.

Genotyping of the edited gene is an important validation for CRISPR edited cells. Specifically, it must be confirmed that the cells carry the same mutation throughout the expansion and culture conditions.

Genomic DNA extraction: 0.5E6 cells were spun down and the supernatant was removed. The cells were resuspended in 50uL Quick Extract buffer. The cells were mixed with buffer thoroughly and the cell-buffer mix was transferred to a PCR tube. Genomic DNA was extracted and subjected to PCR using the SuperFi55 program in a Thermocycler. C1F-P2 cells were used as the wild-type control for comparison of the resulting sequences. Gel electrophoresis was performed to confirm the amplification of the PCR products. Clearly identifiable PCR products were observed from all reactions. The PCR products were also analyzed by Sanger sequencing. Forward primers were used for the sequencing primer.

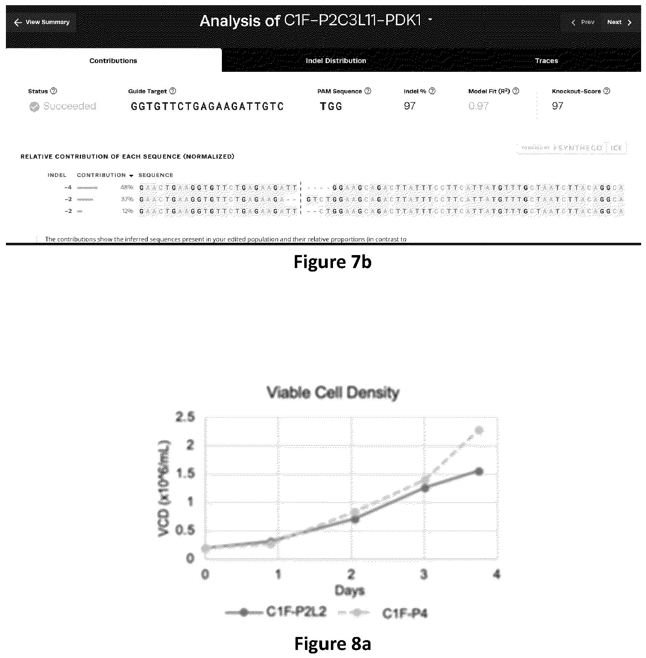

The results of the Sanger sequencing were downloaded into the Synthego ICE analysis tool for analysis. As shown in Figures 7a-7b, C1F-P4 maintained the same edits as observed in the first generation: -2bp for LDHA and -2/-4 bp for PDK1.

### Western Blot Analysis

Western blot analysis was used to confirm the protein expressed by the knocked-out genes is not expressed.

Cell pellets were collected at different stages (adherent cell banks, suspension adaptation and during growth curve studies). 2E+06 cells were spun down for each pellet.

Cell lysates were prepared. The lysate buffer used was 1x RIPA buffer (G Bioscience 786-490) mixed with 100x Halt protease & phosphatase inhibitor (Fisher Scientific PI78442). The buffer was put on ice to cool. The cell lysate was prepared by resuspending the cells in 200uL lysate buffer and mixed well. The cell lysate was put on ice for 30 minutes to cool.

40uL Laemmli SDS sample buffer (6X, Fisher Scientific AAJ6137AD) was added to the cell lysate in an Eppendorf tube and mixed well. The Eppendorf tube was placed in 98°C heat block for 10 minutes. Samples can be used immediately SDS-PAGE runs or stored at -80°C for future usage.

SDS-PAGE was performed by preparing 1X running buffer. The SDS precast gel was assembled in the Criterion Cell tank and running buffer was loaded inside (sample loading area) and outside of the gel in the tank. 10uL of PageRuler protein ladder and 15uL of sample were loaded into each well. The gel was run to resolve protein on the gel.

To transfer the gel, a transfer buffer was prepared. The nitrocellulose membrane was rinsed with transfer buffer for 5 minutes. The transfer sandwich was assembled in the Trans-blot turbo cassette. Mix-range transfer was used for the transfer (2.5A constant, up to 25V, 7 minutes). The membrane was removed from Trans-blot cassette. The membrane was washed three (3) time with TBST. The membrane was immersed in TBST for 5 minutes.

The TBST was removed and five (5) mL of BLOTTO blocking buffer was added to the membrane. The membrane was immersed in the BLOTTO blocking for 15 minutes.

The primary antibody (Anti-LDHA and Anti-PDK1) was diluted (1:1000) in BLOTTO blocking buffer and incubated overnight (4°C or room temperature for >2 hours). The primary antibody was removed from the BLOTTO blocking buffer and washed with TBST (quickly washed 3 times and washed 3 times for 5 minutes each time).

The secondary antibody (Donkey anti rabbit secondary antibody, HRP conjugated) was diluted (1:1000) in BLOTTO blocking buffer and incubated at room temperature for 1 hour. The secondary antibody was removed and washed with TBST (quickly washed 3 times and washed 3 times for 5 minutes each time).

ECL reagents (A and B) were warmed to room temperature. The membrane was removed and placed on a piece of plastic wrap and lightly tapped with a Kimwipe to remove excess buffer. ECL reagents A and B were mixed and applied on top of the membrane. Plastic wrap was used to sealed to ensure the entire membrane is immersed in ECL reagents. The membrane was imaged in ChemiDoc. β-Actin was used as the internal control.

The membrane was washed with TBST three (3) times and β-Actin antibody was added and incubated for 1 hour (1:5000). β-Actin antibody was removed and washed with TBST (quick wash 3 times and washed 3 times for 5 minutes each time). The ECL reagents were again added and the membrane was imaged using ChemiDoc.

C1F-P2 cells expressed LDHA and PDK1 proteins. Protein bands for LDHA and PDK1 were not observed in the C1F-P4 samples. The results confirmed C1F-P4 was a double knockout of LDHA and PDK1.

### LDH Activity Assay

LDH enzyme activity can be measured by an LDH activity assay. To validate the LDH activity in the C1F-P4 cells, LDH activity in the cell lysate was measured. Cell pellets of C1F-P2L2 (maintenance pellet) and C1F-P4 (growth curve day 1) were used for the assay.

Cell pellets were collected and stored in -80°C freezer. The cells were lysed in radioimmunoprecipitation assay (RIPPA) lysis buffer to prepare the cell lysates. The lysate volume was normalized to 1 million cells/mL based on the cell count during pellet collecting. The lysate was incubated on ice for 30 minutes and then the samples were prepared. The results are shown in Table 3.

**Table 3. LDHActivity**

| **Cell Line** | **C1F-P2L2** | **C1F-P4** |
|---|---|---|
| LDH Activity (1 Mc/mL) | 961.69 | 58.04 |

### Example 2. Performance Evaluation of C1F-P4 Cells

### Well-plate growth curve analysis

Plate suspension cultures of C1F-P4 as well as C1F-P2L2 cells were prepared. Cells were seeded 0.2E+06 cells/well in the middle 8 wells of a 24-well plate with 1mL DF-10 media. Two wells were sacrificed daily to perform ViCell and Nova reading to track cell growth and metabolites.

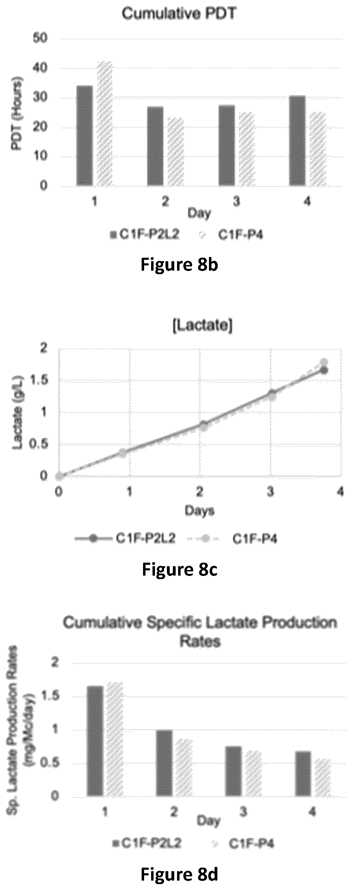

As shown in Figures 8a and 8b, C1F-P4 growth surpassed C1F-P2L2 throughout the experiment and exhibited lower cumulative population doubling time (PDT). At day 4, C1F-P4 showed 46% increase in VCD and 17.5% reduction in PDT. Total lactate amount in the media was comparable in both cell lines, but since C1F-P4 achieved much higher cell number, the specific lactate production rate was reduced ~16% (Figures 8c and 8d). C1F-P4 outperformed C1F-P2L2.

### Suspension culture growth curve analysis

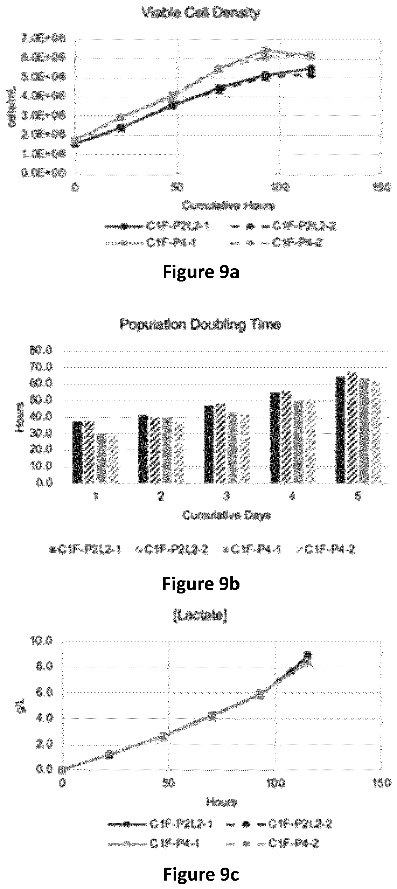

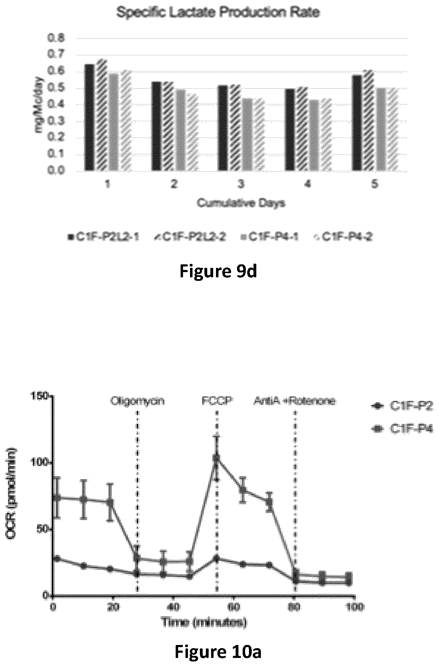

Suspension growth curves were performed to evaluate the growth and metabolic performance of C1F-P4 cells in shake flasks. On day 4 of the growth curve, C1F-P4 reached higher VCD while exhibiting lower cumulative specific lactate production. This suggests improved lactate production and that the cells might be able to extend the proliferation period compared with the same conditions in C1F-P2L2 cells. The results are shown in Figures 9a-9d.

The growth of C1F-P4 is faster than C1F-P2L2 cells. A similar lactate accumulation in the media results in a specific lactate production rate of ~13.5% less than C1F-P2L2 cells. From two independent experiments, it is concluded that the C1F-P4 cells showed promising growth performance and improved lactate production rates. Reduced specific lactate production rate is important for scale up bioreactor runs as lower levels of waste byproducts should improve overall cell performance and extend the bioreactor run time.

### Seahorse extracellular bioenergetic flux analysis

To support ongoing cellular processes, energy is utilized in cells in the form of ATP. Cellular energy is produced in cells from either cytoplasmic glycolysis or mitochondrial oxidative phosphorylation, processes termed cellular metabolism. In addition to the generation of cellular energy, cellular metabolism can also generate waste by-products that can negatively impact growth. Seahorse metabolic flux analyzer can concurrently measure oxygen consumption rate (OCR, surrogate measure for oxidative metabolism), extracellular acidification rate (ECAR, surrogate measure for glycolytic metabolism), as well as ATP production rate of cells. Through these measurements the cellular respiratory profile and glycolytic activities of cells can be understood.

To compare the metabolic profile of C1F-P2, C1F-P2L2 and C1F-P4 cells, a mitochondrial stress test on the Seahorse metabolic flux analyzer was performed. Cells were plated into the 96-well assay plate and refreshed with Seahorse assay media the next day and loaded into the Seahorse extracellular flux analyzer. It will first measure the basal respiration rate of cells. Oligomycin is then added into each sample to inhibit ATP synthase, and the OCR value difference between basal level and after oligomycin treatment represented the ATP-linked respiration rate. Cells are then treated with Carbonyl cyanide-4 (trifluoromethoxy) phenylhydrazone (FCCP) to collapse all proton gradient and result in maximum oxygen consumption. Cells then treated with complex I inhibitor rotenone and complex III inhibitor antimycin to block all mitochondrial respiration activity.

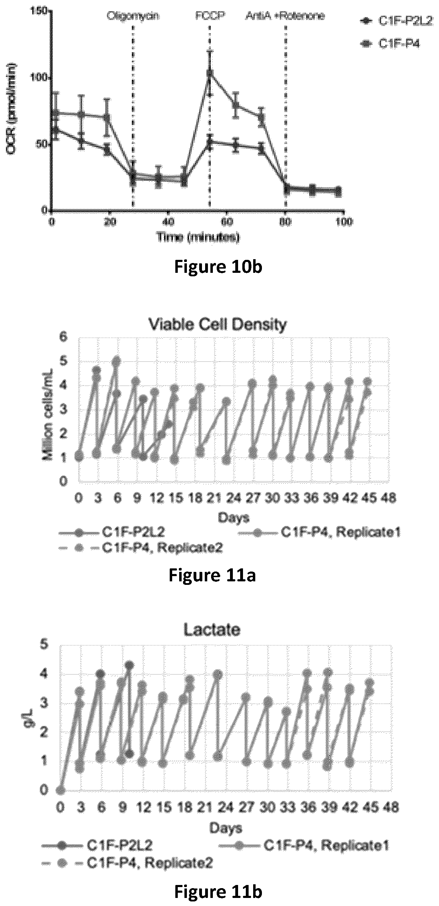

The OCR data were shown in Figure 10. Compare with C1F-P2 or C1F-P2L2 cells, C1F-P4 showed higher basal respiration rate as well as higher maximum oxygen consumption rate. This result suggested that C1F-P4 cells indeed showed improved oxygen consumption profile. the ratio of ATP generated from oxygen consumption or glycolysis was then calculated. As shown in Table 4, it was found that 93.82% of ATP were generated from glycolysis in C1F-P2 cells, indicating that C1F-P2 cells are predominately using glycolysis to generate energy. However, glycolysis generated ATP were decreased to 74.86% in C1F-P4 cells, suggesting that C1F-P4 indeed showed improved metabolic profile by switching partially switching energy production through TCA cycle in mitochondria. Similar metabolic improvement in C1F-P2L2 cells was also observed, which is consistent with the improved cell growth performance and metabolic profile compared with C1F-P2 cells.

In brief, Seahorse metabolic analyzer further confirm that C1F-P4 showed improved metabolic activity compared to C1F-P2 or C1F-P2L2.

**Table 4. ATP rate information from Seahorse analyzer**

| Groups | **XF ATP Rate Index** | **% Glycolysis** | **% OXPHOS** |
|---|---|---|---|
| C1F-P2 | 0.07 | 93.82 | 6.18 |
| C1F-P2L2 | 0.32 | 75.97 | 24.03 |
| C1F-P4 | 0.34 | 74.86 | 25.14 |

### Seed train process development to achieve higher cell densities

For future high-production, large-scale manufacturing of C1F-P4 cells, it is necessary to develop a consistent seed train process capable of achieving higher peak cell densities with batch-culture and split passaging. In a standard-density seed train, C1F-P2L2 and C1F-P4 cells generally achieve a peak cell density four-fold increased from the inoculum density. Given C1F-P4 showed improved growth and more a stable metabolic profile in seed train culture relative to C1F-P2L2, it was hypothesized that C1F-P4 cells could be maintained with a higher inoculum density (1 million cell/mL) to achieve a larger final cell density (4± 0.5 million cells/mL) in a continuous seed train process. The medium was modified to account for the metabolic needs of a high-density culture, utilizing a DMEM/F-12 basal media with increased sodium bicarbonate and glucose, as well as 1% (v/v) of a concentrated amino acid supplementation feed. Replicate cultures of C1F-P4 and C1F-P2L2 were inoculated in parallel targeting 1 million cells/mL and were maintained by split passaging every 3-4 days using a ratio of 1 part cell culture to 3 parts fresh media.

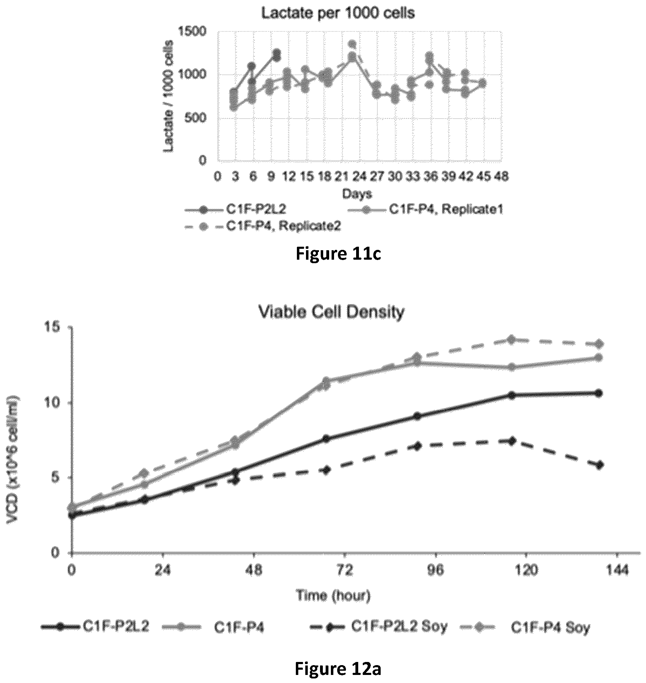

As shown in Figures 11a-11c, C1F-P2L2 cells reached >4 millions on day 3 of the initial passage, but the VCD count gradually decreased over the subsequent 3 passages. By the fourth passage, C1F-P2L2 cells were only able to achieve 2 million cells/mL by day 3 and 2.5 million cells/mL by day 4, suggesting that a higher density seed train process is not feasible for C1F-P2L2. C1F-P2L2 cultures were terminated after the 4^{th} passage. On the contrary, C1F-P4 cells performed similarly to C1F-P2L2 cell in the first passage but were consistently able to achieve ~4 million cells/mL peak density for 14 continuous passages without substantial accumulation of lactate over time. These results demonstrate that C1F-P4 cells are a more preferred candidate for a high-production manufacturing process.

### Example 3. C1F-P4 performance in AMBR250 bioreactor

### C1F-P4 performance in an AMBR250 bioreactor production run

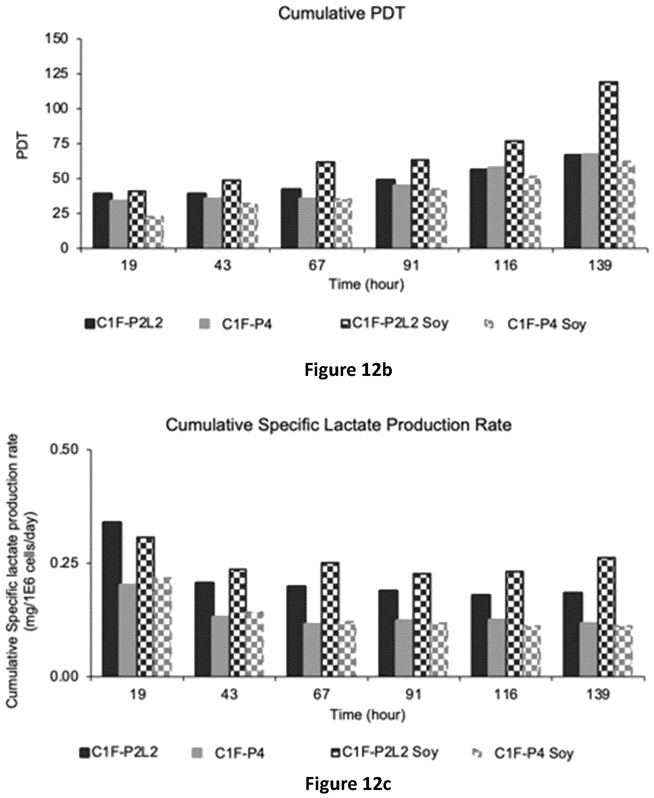

To evaluate the performance of C2F-P4 in an AMBR250 bioreactor, the cells were scaled up and seeded in the bioreactor. Briefly, cells were seeded at 2.5e+06 cells/mL and glucose was slowly fed during the run. The effect of soy hydrolysate was evaluated since it showed growth advantages in parallel work. As shown in Figure 12a-12b, C1F-P4 outperformed C1F-P2L2 cells in both tested conditions. With the addition of soy hydrolysate, C1F-P4 reached 14.16 e+06 cells/mL VCD and 31.09 g/L wet cell weight, a 33% increase in VCD and 53% increase in wet cell weight compared with C1F-P2L2 control cells. C1F-P4 also showed reduced (57.6% lower than C1F-P2L2) cumulative specific lactate production rate (Figure 12c). Overall, C1F-P4 showed strong growth performance and improved lactate production rate.

### Continuous passage of C1F-P4 cells cultured in AMBR250 bioreactors

To achieve cell densities above 10e+06 cells/mL in bioreactors, a fed-batch process has been employed that typically starts with a seed density of 2.5 e+06 cells/mL achieved by a spin passage. In a large-scale production process, however, spin passaging in not feasible. Therefore, it is necessary to select a cell line capable of achieving 10e+06 cells/mL by day 3 to be used as inoculum following a 1:4 split passage for subsequent stages of cell passage.

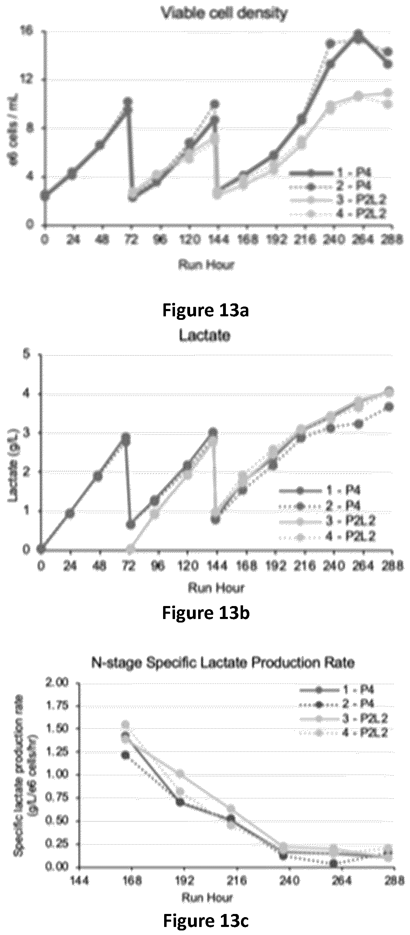

Cell lines C1F-P2L2 and C1F-P4 were therefore evaluated for consecutive passaging performance in AMBR250 bioreactors using a serum-free media formulation supplemented with soy hydrolysate, amino-acid supplement, and insulin. Four AMBR were inoculated at a density of 2.5 million cells/mL. As shown in Figure 13, C1F-P4 were able to achieve three continuous passages in AMBR250 bioreactors. At N-1 stage, C1F-P2L2 reached VCD of 7 million cells/mL on day 3, but C1F-P4 reached ~ 9 million cells /mL. Following passaging into the final (N) stage bioreactor, C1F-P4 consistently outperformed C1F-P2L2 and reached a peak VCD of 15 million cells/mL (Table 6). C1F-P4 also achieved final harvest wet cell weight of 25 g/L, a 31.6% improvement compared with C1F-P2L2 cells (18.99 g/L). These data demonstrate the capacity of the C1F-P4 cell line for high cell density bioreactor production.

**Table 6. Performance of C1F-P4 and C1F-P2L2 in AMBR250 reactor**

| N-stage | C1F-P4 | C1F-P2L2 |
|---|---|---|
| Peak VCD (e6 cells/mL) | 15 | 10 |
| Wet cell weight (g/L) | 25.77 | 18.99 |

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize or be able to ascertain using no more than routine experimentation, many equivalents to the specific embodiments of the invention described herein. Such equivalents are intended to be encompassed by the following claims.
The invention provides the following numbered embodiments:
1. A method for producing cell biomass for animal dietary consumption, the method comprising:
   (a) modifying one or more cell populations to decrease production of one or more chemicals that adversely affect cell proliferation, cell viability, or both;
   (b) cultivating cells from the modified cell populations in a batch or fed-batch process to obtain a cultivated cell mass; and
   (c) harvesting the cultivated cell mass for cultivated meat production.
2. The method of embodiment 1, wherein the one or more chemicals is lactate.
3. The method of embodiment 1 or 2, wherein the one or cell populations are modified by inactivating one or more genes that encode for lactate production in the one or more cell populations.
4. The method of any one of embodiments 1-3, wherein the gene is inactivated via a mutation, deletion, addition, or combinations thereof in a nucleotide sequence.
5. The method of any one of embodiments 1-3, wherein the gene is inactivated by one or more techniques selected from homologous recombination, CRISPR-Cas9, TALENs, and combinations thereof.
6. The method of any one of embodiments 1-5, wherein one gene is inactivated (single knockout).
7. The method of any one of embodiments 1-5, wherein two genes are inactivated (double knockout).
8. The method of any one of embodiments 1-5, wherein the gene is selected from the group consisting of lactate dehydrogenase A (LDHA) gene having the sequence of NCBI Gene ID 396221, pyruvate dehydrogenase kinase 1 (PDK1) gene having the sequence of NCBI Gene ID 425972 , and combinations thereof.
9. The method of embodiment 6, wherein both LDHA and PDK1 are inactivated.
10. The method of embodiment 7, wherein the percent deactivation is at least 50%, 55%, 60%, 65%, 70%, 75%, 80%, 85%, 90%, 91%, 92%, 93%, 94%, 95%, 96%, 97%, 98%, or 99%.
11. The method of any one of embodiments 1-10, wherein the one or more cell populations are selected from the group consisting of fibroblasts, muscle cells, myosatellite cells, myoblasts, pre-adipocytes, adipocytes, epithelial cells, or combinations thereof.
12. The method of embodiment 11, wherein the one or more cell populations is a fibroblast cell population from a species selected from *Gallus gallus, Melagris gallopavo, Anas platyrhynchos, Bos taurus, Sus scrofa, Ovia aries, Salmo salar, Thunnus thynnus, Gadus morhua, Homarus americanus, Litopenaeus setiferus, Oncorhynchus mykiss, and Oreochromis niloticus.*
13. The method of embodiment 11, wherein the one or more cell population is an epithelial cell population from *Bos tauris.*
14. The method of embodiment 13, wherein the epithelial cell population is a kidney cell population.
15. The method of any one of embodiments 1-14, further comprising maintaining a master cell bank of the one or more cell populations modified to decrease production of one or more chemicals that adversely affect cell proliferation, cell viability, or both.
16. The method of embodiment 15 further comprising:
   (a) expanding selected cell populations from the master cell bank;
   (b) cryopreserving and storing selected cell populations in a master cell bank stock inventory;
   (c) seeding and cultivating cells from the master cell bank stock inventory in a culture medium; and
   (d) harvesting the cultivating cell biomass for animal dietary consumption.
17. A cultivated cell biomass for animal dietary consumption obtained by the method of any of embodiments 1-16.
18. A cultivated meat food product comprising one or more cell populations produced by any of the methods of embodiments 1-16.
19. A cultivated meat food product comprising the cultivate cell biomass of embodiment 18.

## Claims

1. A method for producing cell biomass for animal dietary consumption, the method comprising:
(a) modifying one or more cell populations to decrease production of one or more chemicals that adversely affect cell proliferation, cell viability, or both;
(b) cultivating cells from the modified cell populations in a batch or fed-batch process to obtain a cultivated cell mass; and
(c) harvesting the cultivated cell mass for cultivated meat production.

2. The method of claim 1, wherein the one or more chemicals is lactate.

3. The method of claim 1 or 2, wherein the one or cell populations are modified by inactivating one or more genes that encode for lactate production in the one or more cell populations;
optionally wherein the gene is inactivated via a mutation, deletion, addition, or combinations thereof in a nucleotide sequence;
optionally wherein the gene is inactivated by one or more techniques selected from homologous recombination, CRISPR-Cas9, TALENs, and combinations thereof.

4. The method of any one of claims 1-3, wherein one gene is inactivated (single knockout) or wherein two genes are inactivated (double knockout).

5. The method of claim 3 or 4, wherein the gene is selected from the group consisting of lactate dehydrogenase A (LDHA) gene having the sequence of NCBI Gene ID 396221, pyruvate dehydrogenase kinase 1 (PDK1) gene having the sequence of NCBI Gene ID 425972 , and combinations thereof;
optionally wherein both LDHA and PDK1 are inactivated.

6. The method of any one of claims 3-5, wherein the percent deactivation is at least 50%, 55%, 60%, 65%, 70%, 75%, 80%, 85%, 90%, 91%, 92%, 93%, 94%, 95%, 96%, 97%, 98%, or 99%.

7. The method of any one of claims 1-6, wherein the one or more cell populations are selected from the group consisting of fibroblasts, muscle cells, myosatellite cells, myoblasts, pre-adipocytes, adipocytes, epithelial cells, or combinations thereof.

8. The method of claim 7, wherein the one or more cell populations is a fibroblast cell population from a species selected from *Gallus gallus, Melagris gallopavo, Anas platyrhynchos, Bos taurus, Sus scrofa, Ovia aries, Salmo salar, Thunnus thynnus, Gadus morhua, Homarus americanus, Litopenaeus setiferus, Oncorhynchus mykiss, and Oreochromis niloticus.*

9. The method of claim 7, wherein the one or more cell population is an epithelial cell population from *Bos tauris.*

10. The method of claim 9, wherein the epithelial cell population is a kidney cell population.

11. The method of any one of claims 1-10, further comprising maintaining a master cell bank of the one or more cell populations modified to decrease production of one or more chemicals that adversely affect cell proliferation, cell viability, or both.

12. The method of claim 11 further comprising:
(a) expanding selected cell populations from the master cell bank;
(b) cryopreserving and storing selected cell populations in a master cell bank stock inventory;
(c) seeding and cultivating cells from the master cell bank stock inventory in a culture medium; and
(d) harvesting the cultivating cell biomass for animal dietary consumption.

13. A cultivated cell biomass for animal dietary consumption obtained by the method of any of claims 1-12.

14. A cultivated meat food product comprising one or more cell populations produced by any of the methods of claims 1-12.

15. A cultivated meat food product comprising the cultivated cell biomass of claim 13.
